(19) Europäisches Patentamt / European Patent Office / Office européen des brevets

(11) EP 2 185 166 B1

(12) EUROPEAN PATENT SPECIFICATION

(45) Date of publication and mention of the grant of the patent:
**11.11.2015 Bulletin 2015/46**

(21) Application number: **08783138.4**

(22) Date of filing: **03.09.2008**

(51) Int Cl.:
*A61K 36/185* (2006.01) *A61K 8/97* (2006.01)
*A61Q 19/08* (2006.01) *A61P 29/00* (2006.01)
*A61P 17/00* (2006.01)

(86) International application number:
**PCT/BR2008/000269**

(87) International publication number:
**WO 2009/030008 (12.03.2009 Gazette 2009/11)**

(54) **PROCESS FOR PREPARING A PLANT EXTRACT OF PASSIFLORA ALATA AND USE OF SAID EXTRACT IN COSMETIC AND PHARMACEUTICAL COMPOSITIONS**

VERFAHREN ZUR HERSTELLUNG EINES PFLANZENEXTRAKTS VON PASSIFLORA ALATA UND VERWENDUNG DIESES EXTRAKTS IN KOSMETISCHEN UND PHARMAZEUTISCHEN ZUSAMMENSETZUNGEN

PROCEDE DE PREPARATION D'UN EXTRAIT VEGETAL DE PASSIFLORA ALATA ET UTILISATION DUDIT EXTRAIT DANS DES COMPOSITIONS COSMETIQUES ET PHARMACEUTIQUES

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **03.09.2007 FR 0706151**

(43) Date of publication of application:
**19.05.2010 Bulletin 2010/20**

(73) Proprietors:
- **Natura Cosméticos S.A.**
  **06882-700 Itapecerica da Serra- SP (BR)**
- **Universidade Federal de Santa Catarina**
  **CEP 88040-900 Florianópolis- SC (BR)**

(72) Inventors:
- **GESZTESI, Jean-Luc**
  **05017-000 Sâo Paulo- SP (BR)**
- **DA LUZ MOREIRA, Patricia**
  **E-13030-210 Campinas- SP (BR)**
- **LORENCINI, Márcio**
  **12910-320 Bragança Paulista- SP (BR)**
- **MANFIO, Gilson Paulo**
  **13094-030 Campinas- SP (BR)**
- **DELARCINA JUNIOR, Sergio**
  **05632-090 - São Paulo - SP (BR)**
- **SOARES ESTEVES, Simone**
  **13214-584 Jundiaí - SP (BR)**
- **CALIXTO, João Batista**
  **88035-120 Florianópolis- SC (BR)**
- **FERRARI, Cintia Rosa**
  **06390-500 São Paulo- SP (BR)**
- **BRAZ, Thiago**
  **E-03470-080 São Paulo - SP (BR)**
- **COLLINA ROMANHOLE, Rodrigo**
  **03120-010 São Paulo - SP (BR)**
- **PEDROSO DE OLIVEIRA, Ana Paula**
  **05021-010 São Paulo- SP (BR)**
- **DE OLIVEIRA, Elaine Cristina**
  **05057-030 São Paulo - SP (BR)**
- **HURTADO MEDINA, Sandra Patricia**
  **F-75016 Paris (FR)**

(74) Representative: **Pearson, Samuel John et al**
**Abel & Imray**
**20 Red Lion Street**
**London WC1R 4PQ (GB)**

(56) References cited:
**EP-A- 1 541 037 US-A- 5 093 109**

- **PETRY R D ET AL: "Comparative pharmacological study of hydroethanol extracts of Passiflora alata and Passiflora edulis leaves." PHYTOTHERAPY RESEARCH : PTR MAR 2001, vol. 15, no. 2, March 2001 (2001-03), pages 162-164, XP002477044 ISSN: 0951-418X**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- VARGAS ET AL: "Passiflora alata and Passiflora edulis spray-dried aqueous extracts inhibit inflammation in mouse model of pleurisy" FITOTERAPIA, IDB HOLDING, MILAN, IT, vol. 78, no. 2, 1 February 2007 (2007-02-01), pages 112-119, XP005868628 ISSN: 0367-326X
- RUDNICKI ET AL: "Antioxidant and antiglycation properties of Passiflora alata and Passiflora edulis extracts" FOOD CHEMISTRY, ELSEVIER SCIENCE PUBLISHERS LTD, GB, vol. 100, no. 2, 3 July 2006 (2006-07-03), pages 719-724, XP005518706 ISSN: 0308-8146
- PEREIRA CINTIA A M ET AL: "A HPTLC densitometric determination of flavonoids from Passiflora alata, P. edulis, P. incarnata and P. caerulea and comparison with HPLC method." PHYTOCHEMICAL ANALYSIS : PCA 2004 JUL-AUG, vol. 15, no. 4, July 2004 (2004-07), pages 241-248, XP002477045 ISSN: 0958-0344

## Description

Field of the Invention

The present invention relates to the use of plant extracts of *Passiflora alata* as an anti-inflammatory and analgesic active ingredient in cosmetic and pharmaceutical compositions.

Background of the Invention

Plant species of the genus *Passiflora (Passifloraceae)* are native of tropical areas and their major compounds are flavonoids. These plant species are known in folk medicine due to their pharmacological properties, such as anxiolytic and hypotensive action, among others. Some plant species of the genus *Passiflora* are also known as vegetable drugs with anti-inflammatory action. Some prior-art documents relating to the preparation of *Passiflora* extracts and their use in cosmetic and pharmaceutical compositions are presented below.

Document WO2005/097153 discloses a specific method for the preparation of passion fruit *(Passiflora)* extracts and the use of such extracts as hepatoprotective agents, antioxidants and anti-inflammatory agents in mammals. However, this document does not refer to particular species of *Passiflora* or to improved characteristics of said species.

Document EP 1 537 789 discloses compositions for topical application comprising carotenoids, tocopherols and passion flower extracts having anti-inflammatory activity.

Document JP 200200336 discloses a cosmetic product comprising a cymbidium extract and a plant extract that inhibits the production of melanin, such as *Passiflora,* and having anti-inflammatory activity.

Document JP 2000159657 discloses a preparation for external and topical application to the skin comprising plant extracts of the genus *Passiflora* having inhibiting activity on the production of melanin and a whitening effect.

Document JP 2001122731 discloses a cosmetic composition containing extracts of *Passiflora antioquiensis* and *Passiflora mollissima* having prolonged hydration activity and significant efficacy against inflammatory diseases.

Document JP 2002332224, in turn, discloses a cosmetic composition to combat skin aging comprising an extract of *Passiflora incarnata L.* having activity on the aging symptoms and comprising a hydration ingredient or a cell activator ingredient.

Document JP 7233044 also discloses a cosmetic product for preventing wrinkles with cell activation action, wherein the composition according to said document comprises a mixture of extracts selected from several plants, including *Passiflora, Pinelliae Tuber and Mori Fructus.*

Document WO 2005/077328 relates to an anti-wrinkle cosmetic product based on plant extracts. The products disclosed in said document are water-in-oil systems containing a *Passiflora* extract and extracts of papaver, mentha and myrtus aiming at achieving a long-lasting moistening effect and improved elasticity.

Document JP 2001226219 discloses a cosmetic composition containing a diluted plant extract, said plant extract being selected from *Passiflora caerulea L.* and *Passiflora edulis Sims,* while document JP 7118139 discloses a cosmetic product promoting a skin beautifying effect comprising a plant extract, such as *Passiflora SSP.*

Document JP 2004155664 relates to an improved skin functions agent for internal use, said skin functions being improved with the use of a vegetable powder of plants from the genus *Passiflora,* among others. Document WO 2005/053435 discloses preparations for oral and/or topical administration containing *Passiflora incarnata* as well as carotene and tocopherols.

The inventors demonstrate in the present application that extracts prepared from plants of the genus *Passiflora* have improved anti-inflammatory and analgesic activity, and they developed an extraction process so as to obtain an extract rich in flavonoids resulting in said improved anti-inflammatory and analgesic activity, directed for topical and/or internal (oral) use.

Brief Description of the Drawings

Figure 1 contains a brief description of the extraction process for obtaining a dry extract from the leaves of *Passiflora alata* enriched in flavonoids and, specifically, in vitexin-2"-O-rhamnoside (CAS 64820-99-1).

Figure 2 presents the chromatographic profile obtained by HPLC (High Performance Liquid Chromatography) of the extract of *Passiflora alata* obtained according to the present invention.

Figure 3 presents the chromatographic profile obtained by HPLC of a fraction of the aqueous extract of *Passiflora alata* fractioned by a preparative chromatography, using silica as the stationary phase. This fraction (Fr13-23) has the glycosylated flavone vitexin-2-0-rhaminoside as its major component. The fractioning was carried out in order to prove the in vitro biological activities of this compound, which is also present in the original aqueous extract.

Figures 4 to 14 depict graphs containing data relating to safety and efficacy tests of samples of *Passiflora alata* extracts according to the present invention.

Summary of the Invention

The present invention relates to the use of plant extracts of *Passiflora alata* as an anti-inflammatory agent in cosmetic and pharmaceutical compositions.

The present invention also relates to a process for obtaining a plant extract of *Passiflora alata,* comprising the steps of:

a) submitting leaves of *Passiflora alata* plants to an extraction with water to obtain an aqueous extract;
b) submitting the aqueous extract obtained in (a) to at least one elution step with a hydroalcoholic solution in a chromatographic column filled with a specific resin this resin consisting of polyvinylpyrrolidone or polystyrene polymers
c) concentrating and spray drying the extract obtained in (b).

DETAILED DESCRIPTION OF THE INVENTION

The present inventors have found that the plant extracts of *Passiflora alata* have improved anti-inflammatory and analgesic activity and are

useful in cosmetic compositions, particularly anti-wrinkle or anti-aging compositions, as well as in pharmaceutical compositions, particularly for topical or oral administration.

In one embodiment of the invention, the plant extract of *Passiflora alata* is obtained by a process comprising the steps of

a) submitting leaves of *Passiflora alata* plants to an extraction with water to obtain an aqueous extract;
b) filtering the extract obtained in (a);
c) submitting the aqueous extract obtained in (b) to a purification process by means of a specific resin this resin consisting of polyvinylpyrrolidone or polystyrene polymers;
d) eluting the extract with an acidified hydroalcoholic mixture;
e) concentrating the product obtained in (d);
f) resuspending the product obtained in (e) in hot ethanol and later cooling;
g) filtering the solution obtained in (f);
h) concentrating the product obtained in (g);
i) drying the product obtained in (h) with the aid of a solid carrier.

According to another aspect of the invention, the extract of *Passiflora alata* is obtained by a process comprising the steps of:

a) submitting leaves of *Passiflora alata* plants to an extraction with water at the ratio of 1 part of plant to 20 parts of hot water, between 60ºC and 80ºC, preferably at 80ºC, for 2 hours;
b) filtering the extract obtained in (a);
c) submitting the aqueous extract obtained in (b) to a purification process by means of a specific resin, this resin consisting of polyvinylpyrrolidone or polystyrene polymers, preferably polystyrene;
d) eluting the resulting extract with a hydroalcoholic mixture, preferably with ethanol at 30% in water, acidified with acetic acid at pH 5.0;
e) concentrating the product obtained in (d) to 30% of total solid material;
f) resuspending the product obtained in (e) in hot ethanol 96ºGL at 60°C and later cooling to 2°C;
g) filtering the solution obtained in (f);
h) concentrating the product obtained in (g) to 20% of total solid material;
i) drying the product obtained in (h) using a Spray Dryer method with inlet temperature of 180 ºC and outlet temperature of 85 ºC with the aid of the "E 1450" carrier (starch sodium octenyl succinate) at the concentration of 1 to 8%, preferably 8%.

The dry extract resulting from the above process contains C-glycosylated flavonoids, mainly orientin, homoorientin, vitexin, isovitexin and vitexin-2"-O-rhamnosíde; and flavones, mainly luteolin. Their structural formulas are described below:

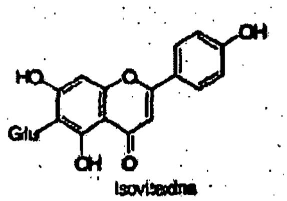

Isovitexina

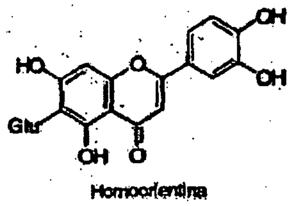

Homoorientina

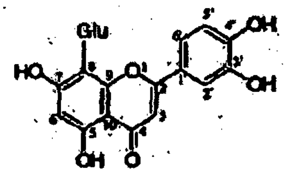

Orientina

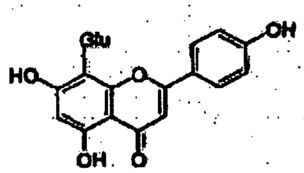

Vitexina

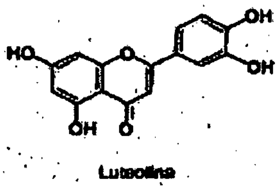

Luteolina

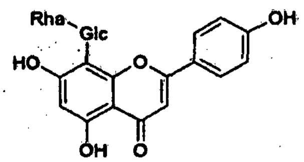

Vitexin-2"-O-rhamnoside

It was found out that the flavonoid present in larger amount in the *Passiflora alata* extract obtained according to the present invention is vitexin-2"-O-rhamnoside (CAS 64820-99-1), this flavonoid only being found in the *Passiflora alata* species when compared with other species of *Passiflora* that are more commonly used as phytotherapeutic agents, being also recommended as a chemical marker to avoid adulterations of the vegetable drug with other species of *Passiflora* (PEREIRA, 2004).

The content of total flavonoids obtained by the present invention is within the range of about 11.40% to about 16.55%, according to the dosing methodology of total flavonoids, such as vitexin, as determined by the Portuguese Pharmacopoeia, 2000.

The content of vitexin-2"-O-rhamnoside obtained by the present invention is about 9.70% to about 11.90%, according to the dosing methodology of this substance by HPLC. The conditions of this analysis were:

- High Efficiency Liquid Chromatography Alliance with PDA 2996 detector, manufacturer Waters.
- Column X-Terra RP18 5um, 4.6 x 150 mm
- Mobile phase: Gradient system composed of Acetonitryl and Formic Acid at 0.2% v/v.
- Flow: 0.6 mL/min
- Temperature: 25 °C
- Volume injected: 20 uL
- Wave length: 340.7 nm

The present invention further relates to cosmetic and pharmaceutical compositions containing plant extract of *Passiflora alata* as an active anti-inflammatory agent. In the compositions according to the present invention, the amount of *Passiflora alata* extract used depends on the final objective intended but preferably it is between 0.25 to 1.00% by weight, based on the total weight of the composition. The compositions of the present invention may contain other ingredients traditionally used in these types of compositions, such as chelating agents, antioxidants, thickening agents, pH adjusting agents, preservatives, moistening agents, conditioning agents, other emollients, filmogenic agents, oleosity adsorbing agents, among others.

The present invention will be illustrated by the examples below.

<u>Example 1 - Obtaining a *Passiflora alata* extract</u>

50g of leaves of *Passiflora alata* were placed in 1,000 L of distilled water and the mixture was heated at 80ºC during 3 hours. The mixture was then passed through a polystyrene column and then eluted with an 30% ethanol solution acidified at pH 5.0 with acetic acid. The resulting extract was filtered and dried.

<u>Example 2 - Obtaining a *Passiflora alata* extract purified by contact with PVPP</u>

100g of dried leaves of *Passiflora alata* were subjected to extraction in distilled water at the ratio of 1:10 (w/v) at 80ºC during 2 hours. After Büchner filtration, 4 aliquots of 50 ml were taken and mixed to 2.5g of PVPP (Divergan F BASF) at 8 ºC.

After 90 minutes under agitation, the samples were eluted in Büchner and PVPP was submitted to the following

eluents for removing the flavonoids contained in the resulting mixture:

a) 50 ml $H_2O$ + $NH_4OH$ 1%, 60ºC, 30 min
b) 50 ml $H_2O$ + HCl 1%, 60ºC, 30 min
c) 50 ml EtOH + $NH_4OH$ 1%, 60ºC, 30 min
d) 50 ml EtOH + Hcl 1%, 60ºC, 30 min

The aliquots were diluted 8.33 times and then analyzed by HPLC in the following analysis conditions:

Mobile phase (A): Formic acid 0.2% in water
Mobile phase (B): ACN
Detection: 337 nm (for flavonoids)
Flow: 0.8 mL/min

Standards: dissolved in methanol: orientin, isoorientin, vitexin, isovitexin, luteolin, apigenin. Concentration of 105 ppm
Column: Phjenomenex Synergil 4 μm Fusion RP-80 150 x 4.6 mm
Gradient:

| Time | % (A) | % (B) |
|---|---|---|
| 0 | 85 | 15 |
| 10 | 85 | 15 |
| 40 | 70 | 30 |
| 50 | 85 | 15 |

The value of the vitexin area was considered for comparison purposes since it is the main flavonoid.
Figure 1 contains a comparative graph between the results obtained for the several samples, and Figure 2 lists chromatograms of several runs performed in this analysis wherein it was noted that PVP is capable of absorbing the glycosylated flavonoids at low temperature.

Example 3 - Obtaining and identifying flavonoids of different extracts of *Passiflora alata*

The flavonoids present in different types of *Passiflora alata* extracts were obtained through 2 different methodologies.

a) Route 1

100g of dried leaves of *Passiflora alata* were suspended in 1000 ml of deionized water. Then, the flavonoids were extracted for 2 hours in a reaction balloon under constant agitation at 3 different temperatures: 60, 70 and 80ºC.
The extract was vacuum-filtered in a Büchner funnel discarding the remainder of leaves retained and completing the volume in the filtrate to new 1000 ml (for each of the different extraction temperatures). From this volume, 500 ml of extract was left to rest overnight in a cold chamber at 5ºC. After 24 hours, the extract was centrifuged at 5ºC and 4100 rpm for 21 minutes, thus obtaining two fractions: precipitate and supernatant. The precipitate was suspended in ethanol, filtered in a common paper filter and the ethanol volume was completed to 80 ml.

b) Route 2

The volume of the other 500 ml of extract separated in route (1) above was precipitated in ethanol twice, after previous cooling at room temperature. Then, the extract was centrifuged at 20ºC and 4100 rpm during 21 minutes, obtaining two fractions: precipitate and supernatant. The precipitate was suspended in ethanol, filtered in a common paper filter and the ethanol volume was completed to 80 ml.

Dosing the concentration of flavonoids

The concentration of the flavonoids contained in the extracts obtained by Routes 1 and 2 was determined

according to Rolim et al, 2005, and the rutin flavonoid was used as standard. The concentration may be seen in Figure 3, where it is expressed in μg/ml.

Figure 3 shows (from the left to the right side) the precipitate of Route 1 suspended in ethanol at 60ºC (R1-60), the precipitate of Route 1 at 70ºC (R1-70), the precipitate of Route 1 at 80ºC (R1-80), the precipitate of Route 2 at 60ºC (R2-60), the precipitate of Route 2 at 70ºC (R2-70) and the precipitate of Route 2 at 80ºC (R2-80). To follow the exact concentrations of the extracts, the results of Figure 3 are also presented in the Table below: Table 1 - Absorbances and respective concentrations of flavonoids of the different extracts of *P. alata*

| Sample | Abs (391 nm) | [] ug/ml |
|---|---|---|
| R1-60 | 0.1908 | 10.10 |
| R1-70 | 0.5093 | 26.78 |
| R1-80 | 0.8166 | 42.87 |
| R2-60 | 0.3397 | 17.90 |
| R2-70 | 0.2791 | 14.73 |
| R2-80 | 0.6023 | 31.65 |

Through the data presented in Figure 3, it is possible to follow the flavonoid concentration profile of all the precipitates suspended in ethanol obtained through Routes 1 and 2 at the three temperatures adopted in the procedures (60, 70 and 80ºC) and it is concluded that the extraction temperature of 80°C was more effective.

Comparing Routes 1 (aqueous) and 2 (with ethanolic precipitation), it is concluded that Route 1 is more effective, showing that the process is optimized in aqueous extraction with subsequent suspension in ethanol. The suspended precipitate from Route 1 at 80ºC achieved a concentration higher than 40 μg/ml.

Example 4 - In vitro cytotoxity and phototoxicity assessment

The objective of this experiment was to determine the cytotoxic and phototoxic effect of the samples of *Passiflora alata* extracts according to the present invention using methodologies established by the National Institute of Health (NIH) EUA, which are internationally valid.

Definitions:

Cytotoxicity: The assay enables to determine the cytotoxic concentration of an active component, when the 3T3 cells are incubated with it for 24 hours. Toxicity is determined in function of the cell viability, monitored by the incubation of the 3T3 cells with Neutral Red, a vital dye which is incorporated by the living cells after 24 hours of contact with the active component (Guidance Document on Using In Vitro Data to Estimate In Vivo Starting Doses for Acute Toxicity. NIH Publication 01-4500 (2001)).

Phototoxicity: The basis for the test is to compare the toxicity of an active component in the presence and absence of a non-cytotoxic dose of UVA radiation. Phototoxicity is measured by the uptake of Neutral Red by the cells that survive incubation with the active component, according to the Borenfreund & Puerner (1985) protocol, which has already been established and standardized by the NIH ("ZEBET/ECVAM/COLIPA Standard Operating Procedure. In Vitro 3T3 Phototoicity Test. 26 Ap, 1998"). Phototoxicity/Photoirritation is defined as a toxic response caused after the first skin exposure to certain products/active components with a subsequent exposure to solar light or which is induced by skin irradiation after the systemic administration of an active component/product ("Spielmann H, Liebsch M, Doring B, Moldenhauer F. First results of an EC/COLIPA validation project of in vitro phototoxicity testing methods. ALTEX. 1994;11(1):22-31").

Material:

3T3 Cells (fibroblast cell line of Balb/C mouse skin).

Reagents:

Dulbecco's Modified Eagle Medium (DMEM), Fetal Bovine Serum (FBS), Neutral Red (SIGMA-ALDRICH), Dimethylsulfoxide (DMSO), Ethanol, Acetic Acid and PBS (Phosphate Buffer Saline).

Methods:

Analysis of solubility and definition of the experiment concentrations: The solubility of the samples is assessed according to essays internationally standardized by NIH. In order to provide better solubilization of the samples in aqueous solutions, such as DMEM (in the cytotoxicity assay) or PBS (in the phototoxicity assay), the use of a maximum concentration of 1% of DMSO or Ethanol is allowed. The concentrations used in the cytotoxicity and photoxicity tests vary according to the results of the sample solubility tests. The maximum concentration tested should not be greater than 3 mg/ml.

Cytotoxicity: The 3T3 cells are plated in a sterile 96-well plate, at a density of $1x10^4$ cells/well. After 24 hours, the cells are incubated during 24 hours with the samples diluted in a culture medium with 5% SFB. Afterwards, they are incubated with Neutral Red during 3 hours. The excess of dye is washed with PBS and the crystals formed are dissolved with Ethanol: Acetic Acid: Water (50:1:49). The absorbance reading is performed at 540 nm and, from the results obtained, the concentration in which 50% of the cells are viable ($CI_{50}$) is calculated. Therefore, the higher the $CI_{50}$ value, the lower the cytotoxic potential of the sample, and vice-versa.

Phototoxicity: The 3T3 cells are plated in a sterile 96-well plate, at a density of $1x10^4$ cells/well. Two identical plates are prepared but one of them will be irradiated with UVA and the other will be kept in the dark, as a control. After 24 hours of plating, the cells are incubated during 1 hour in the dark with the diluted PBS samples. After that, one of them is irradiated with 5 Joules/$cm^2$ of UVA light during 50 minutes. The plates are washed with PBS and receive a culture medium with 10% fetal bovine serum for the recovery period which lasts approximately 12 hours. On the second day of the experiment, the cells are incubated with a Neutral Red solution for 3 hours. The excess of dye is washed with PBS and the crystals formed are dissolved with Ethanol: Acetic Acid: Water (50:1:49). The absorbance reading is performed at 540nm and, from the results obtained, the concentration in which 50% of the cells are viable ($CI_{50}$) is calculated in each plate. The ratio between the two values is the Photo Irritation Factor (PIF), which classifies the sample as phototoxic or not.

Calculation of PIF:

$$PIF = \frac{IC_{50}\ (-U)}{CI_{50}\ (+UV)}$$

When PIF is greater than or equal to 5, the sample is phototoxic.

If it is not possible to obtain the $CI_{50}$ value until the maximum concentration tested in the absence of UV, while in the presence of UV it was possible to observe phototoxic action and calculate $CI_{50}$, >PIF is calculated by dividing the maximum concentration tested by the $IC_{50}$ of the phototoxicity curve with UV:

$$>PIF = \frac{Cmax\ (-UV)}{CI_{50}\ (+UV)}$$

When >PIF is greater than 1, the sample is phototoxic.

<u>Example 4.1</u>

The following samples of *Passiflora alata* extract according to the present invention have been analyzed for cytotoxicity:

Passiflora A = 16.54% flavonoids; 11.02% vitexin-2-o-r
Passiflora B = 11.49% flavonoids; 0.39% vitexin-2-o-r

Firstly, a stock solution of the sample was prepared in PBS at 30 mg/ml containing 10% of DMSO. In the case of cytotoxicity, the stock solution was diluted 10 times in DMEM containing 5% of SFB, obtaining the maximum concentration used of 3 mg/ml containing 1% of DMSO. The other dilutions tested for cytotoxicity have also been prepared with DMEM containing 5% of SFB.

Data relating to the cytotoxicity essay for the samples described in the present application have been used for building the graphs of Figures 4 and 5.

The samples tested and their respective in vitro cytotoxic potentials are indicated in Table 2. Sample 550 was considered cytotoxic in vitro above 0.2 mg/ml.

Table 2: Data obtained in the cytotoxicity test

| Samples | Record | $CI_{50}$ (mg/ml) |
|---|---|---|
| Passiflora Extract | A | NA |
| Passiflora Extract | B | 2,0 |

<u>Example 4.2</u>

The following samples of *Passiflora alata* extract according to the present invention have been analyzed for phototoxicity:

Passiflora A = 16.54% flavonoids; 11.02% vitexin-2-o-r
Passiflora B = 11.49% flavonoids; 0.39% vitexin-2-o-r

Firstly, a stock solution of the sample was prepared in PBS at 30 mg/ml containing 10% of DMSO. In the case of phototoxicity, the stock solution was diluted 10 times in PBS obtaining the maximum concentration used of 3 mg/ml containing 1% of DMSO. The other dilutions tested for phototoxicity have also been prepared with PBS. The samples were filtered in membranes of 0.22 μm.

Data relating to the phototoxicity essay for the samples described in the present application have been used for building the graphs of Figures 6 and 7.

The samples tested and their respective in vitro phototoxic potentials are indicated in Table 3. Samples A and B were considered non-phototoxic in vitro.

Table 3: Data obtained in the phototoxicity test

| Sample | Record | PIF |
|---|---|---|
| Passiflora Extract | A | NA |
| Passiflora Extract | B | NA |

<u>Example 5 - Assessment of the mutagenic potential through reverse mutation test in *Salmonella typhimurium* (Ames test)</u>

The AMES test (Maron & Ames, 1983; OECD Guidelines, 1997) aims at assessing the potential of certain chemicals to induce mutations in the genome of *Salmonella typhimurium* strains through reverse mutation of his- to his+, with and without a metabolic activation system. In this study, it was observed that the *Passiflora alata* extracts do not have mutagenic activity potential in *Salmonella typhimurium* strains.

Example 5.1

The following samples of *Passiflora alata* extract according to the present invention have been used:

Sample C (content of total flavonoids 16.46% and content of vitexin-2-o-r 11.87%)
Sample F (content of total flavonoids 16.10% and content of vitexin-2-o-r 9.67%)
Materials: TA97a, TA98, TA 100, TA102 and TA1535 and TA1537 Strains
Reagents: Sodium azide, 2-nitrofluorene; 9-aminoacridine, cumene hydroperoxide, 2-aminoanthracene, minimum glycosylated medium, top agar, S9 fraction (Molecular Toxicology Incorporated, EUA), deionized water, 4-nitroquinoline-1-oxide, mitomycin C

Test Performance

0.1 ml of overnight-grown bacteria culture and 0.1ml of samples tested was added to each tube containing 3 ml of top agar previously conditioned in dry broth at 45°C. In the tests with metabolic activaction, 0.5 ml/plate of fraction

S9 that has a concentration of proteins of 39.7 mg/ml was added

Before the test, the genotypes of the strains were assessed with the objective of ensuring the original genetic characteristics of the bacteria. By means of selective growth medium, the dependence of the strains on histidine and biotin was analyzed. The presence of *uvrB* deletion was detected by the sensitivity to the ultraviolet light irradiated to the plates. The *rfa* mutation, which increases the permeability of the bacterial wall to the entry of substances to be tested, was detected through the sensitivity to crystal violet. Testing the resistance to ampicillin, the presence of plasmid pKM101 was noted, acting at the DNA repair level, increasing the chemical and spontaneous mutagenicity. The presence of plasmid pAQ1 was verified because it imparts resistance to tetracyclin. The number of spontaneous revertant colonies per plate in each strain was compared at the acceptable rate described in the literature by Maron & Ames (1983).

Positive and negative controls have been included in all the assays. As a negative control, the solvent selected for sample solubilization (100 μl/plate deionized water) was used in order to obtain the number of spontaneous revertant colonies per plate for later comparison with the number of revertants observed in the presence of the samples. As positive controls, known mutagenic substances were used to ensure the response capability of each strain to the mutagen and the efficacy of the metabolic activation system (sodium azide, 4-nitroquinoline-1-oxide, 9-amidoacridine, mitomycin C, 2-aminoanthracene, 2-nitrofluorene, cumene hydroperoxide).

A preliminary test was performed with the TA100 strain to determine the most suitable interval of sample concentration for the definitive test using concentrations 8; 40; 200; 1000 and 5000 μg/plate. The concentrations used in this test have not presented toxicity for the growth of *Salmonella typhimurion.* Therefore, the concentrations of the definitive test have varied from 0.001 to 5 mg/plate. All the concentrations were tested in triplicate in the absence and presence of metabolic activation. The negative and positive controls were performed in triplicate.

The results were expressed in numbers of revertant colonies per plate and by the mutagenicity ratio (MR), corresponding to the ratio between the number of revertant colonies in the test plates and the number of revertant colonies in the negative control plates. The number of spontaneous revertant colonies in each strain was compared at the acceptable normal rate described in the literature (Maron & Ames, 1983).

A result is considered to be positive when the average number of revertant colonies in the test plates is greater than or equal to twice the one observed in the negative control plates (MR >2) for strains TA97a, TA98, TA 100 and TA 102. For strains TA1535 and TA1537, MR should be greater than or equal to three.

The positive controls have presented a mutagenicity ratio between 5 and 152, showing to be suitable and validating the experiment. No significant increase in the number of revertants was observed in the strains tested after the treatment with C and F samples in any one of the concentrations tested in the presence and absence of metabolic activation. Samples C and F showed a mutagenicity ratio of less than 2.

<u>Example 6 - Assessment of the potential for irritation and skin sensitization in vivo</u>

The *Passiflora alata* extract was submitted to an in vivo safety test, according to Fisher, 1995:

Sample C - (content of total flavonoids 16.46% and content of vitexin-2-o-r 11.87%) at 25% and 50% of an aqueous solution.

The extract was submitted to primary irritation and accumulated irritation survey and patch test sensitization. The primary irritation test is assessed with the removal of the patch test after 48 hours of application, with readings performed in 30 minutes and 24 hours after its removal. The accumulated irritation test and the sensitization test, on the other hand, require the application of a patch test on a daily basis for a period of 14 days, followed by a period of rest of 14 days and later reapplication of a patch test for 48 hours, in an area which had not been previously used for assessment of primary irritation.

The study was a randomized, mono-blind, controlled clinical trial. Fifty six volunteers completed the study and no adverse reactions were detected (erythema, edema, papules or vesicles) in the areas of the sample application.

<u>Example 7 - Assessment of the anti-inflammatory potential in human fibroblasts in vitro</u>

The objective of this test was to assess the anti-inflammatory potential of in vitro extracts, using human dermal fibroblast cultures.. The statistical significance ($p < 0.05$) of the data was verified by the analysis of variance (One-Way ANOVA) followed by a Tukey multiple comparison using the SYSTAT 10 software.

<u>Example 7.1</u>

The following samples of *Passiflora alata* extract according to the present invention have been used:

Sample A (content of total flavonoids 16.54% and content of vitexin-2-o-r 11.02%)
Sample B (content of total flavonoids 11.49% and content of vitexin-2-o-r 0.39%)

Human fibroblasts derived from the dermis were incubated with lipopolysaccharide (LPS) of *Escherichia coli* to induce the secretion of pro-inflammatory citokines IL-6 and IL-8 (Interleukin 6 and 8). The anti-inflammatory potential of the sample was identified by the reduction in the secretion of the pro-inflammatory markers.
Material used: Dermis-derived human fibroblasts, kept in culture until the tenth passage at most. Sterile-culture plates with 96 wells, non-sterile plates with 96 wells, conical tubes of 15ml and 50 ml
Reagents: Dulbecco's Modified Eagle Medium (DMEM), Fetal Bovine Serum (FBS), Neutral Red, Dimethylsulfoxide (DMSO), development buffer (ethanol: acetic acid: water, 50:1:49), PBS (Phosphate Buffer Saline), Washing Buffer (PBS containing 0.05% Tween-20), sensitization buffer (0.1 M sodium carbonate), blocking buffer (PBS containing 5% of bovine serum albumin), bacterial lipopolysaccharide (LPS), dexamethasone, hydrocortisone, kits for dosing IL-6 and IL-8 by ELISA (R&D Systems®), TMB substrate development kit (BD Pharmingen®) and stop solution (2N of sulfuric acid).

Cell culture preparation

96-well plates were prepared with $2x10^4$ cells/well in 100 μl of DMEM containing 10% SFB. The cells were incubated overnight in a $CO_2$ oven. The culture medium was discarded and the wells were washed with 200μl of PBS.

Cell stimulation with LPS

100 μl of DMEM containing 1% SFB and the suitable stimuli were added. Incubation was carried in a $CO_2$ oven overnight. The treatments were:

- Control (only DMEM containing 1% SFB);
- LPS 10 ng/ml;
- Dexamethasone 5 uM;
- LPS 10 ng/ml + Dexamethasone 5 uM;
- Extract A 0.1 mg/ml;
- LPS 10 ng/ml + Extract A 0.1 mg/ml;
- Extract A 1 mg/ml;
- LPS 10 ng/ml + Extract A 1 mg/ml;
- Extract B 0.1 mg/ml;
- LPS 10 ng/ml + Extract B 0.1 mg/ml;
- Extract B 1 mg/ml;
- LPS 10 ng/ml + Extract B 1 mg/ml;

Collection of supernatant and analysis of cell viability

After stimulation, supernatants were collected, stored at -20ºC and analysed by ELISA (described below). In order to keep cell viability after stimulation, the cells were incubated with a Neutral Red solution for 3 hours and after washing with 200 μl of PBS, 200 μl of development solution were added, and the reading was performed at 540 nm. In all treatments, the minimum cell viability should greater than or equal to 80% of the control viability. Cell viability data were also used later for normalizing the data obtained by ELISA.

ELISA (Enzyme-Linked Immunosorbent Assay)

For the ELISA test, 96-well plates were used. The quantification of pro-inflammatory markers was determined from the comparisons with the standard curves specifically constructed for IL-6 or IL-8. Culture supernatants were used according to the following steps:

- Addition of the capture antibody diluted in PBS (1:180, 100 μl/well);
- Overnight incubation at room temperature;
- Plate washing 3 times with washing buffer (200 μl/well);
- Blocking of the free sites in the plate with a blocking buffer (200 μl/well);
- Incubation for 1 hour at room temperature;
- Plate washing 3 times with washing buffer (200 μl/well);
- Preparation of the dilutions of the standard and samples with the blocking buffer (1:200);

- Addition of the dilutions of the standard, the samples and the control (100 μl/well);
- Incubation for 2 hours at room temperature;
- Plate washing 3 times with washing buffer (200 μl/well);
- Addition of detection antibody diluted in a blocking buffer (1:180 each, 100 μl/well);
- Incubation for 2 hours at room temperature;
- Plate washing 3 times with washing buffer (200 μl/well);
- Addition of the conjugate of streptoavidin-peroxidase diluted in a blocking buffer (1:200, 100 μl/well);
- Incubation for 20 min at room temperature in the dark;
- Plate washing 3 times with washing buffer (200 μl/well);
- Addition of the substrate solution (100 μl/well);
- Incubation of the plate for 20 min at room temperature in the dark;
- Addition of the stop solution (50 μl/well);
- Absorbance reading at 450nm.

From the results obtained, Tables 2 and 3 were prepared with the secretion inhibition values of IL-6 and IL-8, respectively. The reference column indicates a sample the data of which was used as basis for calculating the inhibition percentages for the other samples. The data is also represented in the graphs of Figures 8 and 9, respectively.

Table 2. Inhibition Rate of IL-6 Secretion.

| Treatment | % Inhibition | Reference |
|---|---|---|
| Dexamethasone | 71 | Control |
| Extract A 0.1 mg/ml | 15 | |
| Extract A 1 mg/ml | 73 | |
| Extract B 0.1 mg/ml | 19 | |
| Extract B 1 mg/ml | 44 | |
| Dexamethasone + LPS | 96 | LPS |
| Extract A 0.1 mg/ml + LPS | 68 | |
| Extract B 1 mg/ml + LPS | 96 | |
| Extract B 0.1 mg/ml + LPS | 69 | |
| Extract B 1 mg/ml + LPS | 85 | |

Table 3. Inhibition Rate of IL-8 Secretion.

| Treatment | % Inhibition | Reference |
|---|---|---|
| Dexamethasone | 68 | Control |
| Extract A 0.1 mg/ml | 34 | |
| Extract A 1 mg/ml | 85 | |
| Extract B 0.1 mg/ml | 55 | |
| Extract B 1 mg/ml | 67 | |
| Dexamethasone + LPS | 53 | LPS |
| Extract A 0.1 mg/ml + LPS | 24 | |
| Extract B 1 mg/ml + LPS | 78 | |
| Extract B 0.1 mg/ml + LPS | 32 | |
| Extract B 1 mg/ml + LPS | 51 | |

According to the data analyzed, samples A and B indicate a potential anti-inflammatory activity, with a reduction in IL-6 and IL-8 secretion in human fibroblasts in vitro ($p < 0.05$). Both extracts have an effect similar to the dexamethasone

used in the model. In general, the extracts used at the concentration of 1 mg/ml showed an inhibition rate very similar or greater than that found for dexamethasone at 5 uM. In addition, this anti-inflammatory effect was also observed in the presence of LPS, a known agent for inducing inflammatory response ($p < 0.05$).

Example 7.2

The following samples of *Passiflora alata* extract according to the present invention have been used:

Sample A (content of total flavonoids 16.54% and content of vitexin-2-o-r 11.02%)
Purified fraction of vitexin-2-0-rhaminosode (Fr13-23)
Dermis-derived human fibroblasts were incubated with lipopolysaccharide (LPS) or UV to induce the secretion of pro-inflammatory markers IL-6 and IL-8. The anti-inflammatory potential of the sample was identified by the reduction in the secretion of the pro-inflammatory markers.
Material used: Dermis-derived human fibroblasts, kept in culture until the tenth passage at most. Sterile-culture plates with 96 wells, non-sterile plates with 96 wells, conical tubes of 15ml and 50ml, radiation chamber with UVB lamp.
Reagents: The same reagents indicated for Example 7.1 were used.

Cell culture preparation

96-well plates were prepared with $2x10^4$ cells/well in 100μl μl of DMEM containing 10% SFB. The cells were incubated overnight in a $CO_2$ oven. The culture medium was discarded and the wells were washed with 200μl PBS.

Cell stimulation with LPS

100μl of DMEM containing 1% SFB and the suitable stimuli were added. Incubation was carried out in a $CO_2$ oven overnight. The treatments were:

- Control (only DMEM containing 1% SFB);
- LPS 10 ng/ml;
- LPS 10 ng/ml + Dexamethasone 5 uM;
- LPS 10 ng/ml + Sample A 1 mg/ml;
- LPS 10 ng/ml + Sample A 0.5 mg/ml;
- LPS 10 ng/ml + Sample A 0.25 mg/ml;
- LPS 10 ng/ml + Sample A 0.125 mg/ml;
- LPS 10 ng/ml + Sample Fr13-23 1 mg/ml;
- LPS 10 ng/ml + Sample Fr13-23 0.5 mg/ml;
- LPS 10 ng/ml + Sample Fr13-23 0.25 mg/ml;
- LPS 10 ng/ml + Sample Fr13-23 0.125 mg/ml.

Cell stimulation with UVB

100 μl of PBS and the suitable stimuli were added. Incubation was carried out in a $CO_2$ oven for 50 min. The treatments were:

- Control (only PBS in cells that were not irradiated);
- UVB 0.23J/cm2 (only PBS with irradiation after incubation);
- UVB 0.23J/cm2 + Hydrocortisone 5 uM;
- UVB 0.23J/cm2 + Sample A 1 mg/ml;
- UVB 0.23J/cm2 + Sample A 0.5 mg/ml;
- UVB 0.23J/cm2 + Sample A 0.25 mg/ml;
- UVB 0.23J/cm2 + Sample A 0.125 mg/ml;
- UVB 0.23J/cm2 + Sample Fr13-23 1 mg/ml;
- UVB 0.23J/cm2 + Sample Fr13-23 0.5 mg/ml;
- UVB 0.23J/cm2 + Sample Fr13-23 0.25 mg/ml;
- UVB 0.23J/cm2 + Sample Fr13-23 0;125 mg/ml.

After the cell irradiation period (except for control), the volumes of the wells were discarded and replaced with the same treatment prepared in 100μl of DMEM containing 1% SFB and the suitable stimuli. Afterwards, CO2 incubation

occurred overnight.

Collection of supernatant and analysis of cell viability

After stimulation, supernatants were collected, stored at -20ºC and assessed by ELISA (described below). In order to keep cell viability after stimulation, the cells were incubated with a Neutral Red solution for 3 hours and, after washing with 200 μl of PBS, 200 μl of development solution were added, and the reading was performed at 540 nm. In all treatments, the minimum cell viability should greater than or equal to 80% of the control viability. Cell viability data was also used later for normalizing the data obtained by ELISA.

ELISA (Enzyme-Linked Immunosorbent Assay)

For the ELISA test, 96-well plates were used. The quantification of pro-inflammatory markers was determined from the comparisons with the standard curves specifically constructed for IL-6 or IL-8. Culture supernatants were used according to the following steps:

- Addition of the capture antibody diluted in sensitization buffer (1:250, 100 μl/well);
- Overnight incubation at 4 ºC;
- Plate washing 3 times with washing buffer (200 μl/well);
- Blocking of the free sites in the plate with a blocking buffer (200 μl/well);
- Incubation for 1 hour at room temperature;
- Plate washing 3 times with washing buffer (200 μl/well);
- Preparation of the dilutions of the standard and samples with the blocking buffer (1:200);
- Addition of the dilutions of the standard, the samples and the control (100 μl /well);
- Incubation for 2 hours at room temperature;
- Plate washing 5 times with washing buffer (200 μl/well);
- Addition of detection antibody and the streptavidin-peroxidase conjugate diluted in a blocking buffer (1:250 each, 100 μl/well);
- Incubation for 1 hour at room temperature;
- Plate washing 7 times with washing buffer (200 μl/well);
- Addition of the substrate solution (100 μl/well);
- Incubation of the plate for 30 min at room temperature in the dark;
- Addition of the stop solution (50 μl/well);
- Absorbance reading at 450 nm.

From the results obtained, Tables 4 and 5 were prepared with the secretion inhibition values of IL-6 and IL-8, respectively, as well as the graphs depicted in Figures 10 and 11 for stimulus with LPS, and 12 and 13 for stimulus with UVB. The reference column indicates a sample whose data were used as basis for calculating the inhibition percentages for the other samples.

Table 4. Inhibition Rate of IL-6 Secretion.

| Treatment | % Inhibition | Reference |
|---|---|---|
| LPS + Dexamethasone | 94 | LPS |
| LPS + Sample A 1 mg/ml | 91 | |
| LPS + Sample A 0.5 mg/ml | 75 | |
| LPS + Sample A 0.25 mg/ml | 62 | |
| LPS + Sample A 0.125 mg/ml | 38 | |
| LPS + Sample Fr13-23 0.125 mg/ml | 84 | |

(continued)

| Treatment | % Inhibition | Reference |
|---|---|---|
| UVB + Hydrocortisone | 71 | UVB |
| UVB + Sample A 1 mg/ml | 85 | |
| UVB + Sample A 0.5mg/ml | 90 | |
| UVB + Sample A 0.25 mg/ml | 91 | |
| UVB + Sample A 0.125 mg/ml | 84 | |
| UVB + Sample Fr13-23 0.125 mg/ml | 80 | |

Table 5. Inhibition Rate of IL-R Secretion.

| Treatment | % Inhibition | Reference |
|---|---|---|
| LPS + Dexamethasone | 82 | LPS |
| LPS + Sample A 1 mg/ml | 90 | |
| LPS + Sample A 0.5 mg/ml | 71 | |
| LPS + Sample A 0.25 mg/ml | 57 | |
| LPS + Sample A 0.125 mg/ml | 28 | |
| LPS + Sample Fr13-23 0.125 mg/ml | 65 | |
| UVB + Hydrocortisone | 79 | UVB |
| UVB + Sample A 1 mg/ml | 86 | |
| UVB + Sample A 0.5 mg/ml | 88 | |
| UVB + Sample A 0.25 mg/ml | 86 | |
| UVB + Sample A 0.125 mg/ml | 74 | |
| UVB + Sample Fr13-23 0.125 mg/ml | 57 | |

According to the data analyzed, samples A and Fr13-23 indicate a potential anti-inflammatory activity, with a reduction in IL-6 and IL-8 secretion in human fibroblasts in vitro ($p < 0.05$).

Sample A showed anti-inflammatory activity in the 2 models (stimulation with LPS and UVB), in all the concentrations tested ($p < 0.05$).

Sample Fr13-23 was not cytotoxic in human fibroblasts at the concentration of 0.125 mg/ml. This is probably due to the fact that this sample has a high concentration of vitexin-2-0-rhaminoside. At this concentration and in the 2 models (stimulation with LPS and UVB), the sample presented considerable anti-inflammatory activity.

Example 8 - Assessment of the expression of adhesion molecules ICAM-1 in vitro.

During the inflammatory process, it is known that the release of TNF-$\alpha$ by macrophages and fibroblasts has an important role in the signaling between leukocytes and endothelial cells. The cell response to lesion comprises the adhesion of leukocytes to the endothelium of post-capillary venules, followed by the migration of the adherent cells to the outside of the vessel, their displacement to the extravascular site and subsequent accumulation on the lesion, mechanisms which have been described as a sequence of complex interactions and signalings between leukocytes and endothelial cells. The increase in the expression of adhesion molecules, such as ICAM-1, V-CAM and E-selectin is a pre-requisite for a stable leukocyte-endothelium adhesion and necessarily precedes the leukocyte extravasation (Quinlan et al., 1999; Fuchs et al., 2001). Therefore, their assessment may be used as an in vitro experimental model for the pro- or anti-inflammatory assessment of new molecules.

Example 8.1

The following sample of *Passiflora alata* extract according to the present invention was used:

Sample A (content of total flavonoids 16.54% and content of vitexin-2-o-r 11.02%)

Human Umbilical Vein Endothelial Cells (HUVEC) were incubated with TNF-α to induce ICAM-1 expression, responsible for cell infiltration in the anti-inflammatory process. The anti-inflammatory potential of the sample was identified by the reduction in the expression of this molecule.
Material: Human Umbilical Vein Endothelial Cells (HUVEC) with a total number of passages of 6
Reagents: Culture medium M199; Fetal Bovine Serum; Trypsin (Gibco). Human TNF-α (Peprotech Mexico, S.A.), human anti-ICAM-1 antibodies (BD Biosciences Pharmingen); phosphate buffer saline.PBS), bovine serum albumin.

Cell culture preparation

24-well plates were prepared with $5x10^4$ cells/well in a culture medium M199 and 10% fetal bovine serum. The cells were incubated in a $CO_2$ oven for 24 hours. After this period, the cells were treated with different concentrations of sample A for 12 hours of incubation. Then, the cells were exposed to 10 ng/ml TNF-α for a period of 6 hours to stimulate the expression of ICAM-1, this time representing the peak of molecule expression in endothelial cells kept in culture, as described by Perfetto et al. (2003) and Jiang et al. (2004). In addition, the concentration of 10 ng/ml TNF-α was determined as being capable of inducing the expression of adhesion molecules without interfering in cell viability (Piela-Smith et al., 1992).
After incubation with TNF-α, the cells were displaced from the plate with trypsin, washed in culture medium with SFB for removing the trypsin contained in the medium, centrifuged during 8 minutes at 300g and resuspended in 1 ml of paraformoldehyde at 1% for 15 minutes for fixing them. Later, the cells were centrifuged again for removing paraformoldehyde, resuspended in 100 μl of PBS with 1% of bovine serum albumin (BSA) and incubated with 5 μl of anti-ICAM antibody (FITC), during one hour at the temperature of 4°C and protected from light. Afterwards, the cells were centrifuged for 8 minutes at 300g and resuspended in PBS for reading the samples in the flow cytometer FACS CALIBUR (BD), connected to an Apple (Machintosh) computer using the Cell QuestPro® software. Ten thousand
(10,000) events were acquired. The marking of the isotypical antibody was used as a negative control for the antibody. The data generated by the cytometer was analyzed with the help of the software, the values being expressed as a percentage of cells marked by the antibody, which indicates the percentage of cells having ICAM-1 surface expression, and also the mean fluorescence intensity of each sample, which quantitatively indicates the expression of adhesion molecules before and after the treatments with TNF-α and the sample at the different concentrations.
The results obtained with the treatment are expressed in the graphs of Figures 14 and 15.
The cells that were incubated only with the higher concentration of the sample (316 μg/ml) presented an increase in the expression of ICAM-1 adhesion molecules in relation to the cells kept only in a culture medium; however, this increase did not show a significant difference ($p > 0.05$). The incubation with TNF-α has caused a significant increase ($p < 0.001$) in the expression of these adhesion molecules. The incubation of cells with a lower concentration of the sample (100 μg/ml) was capable of partially (although significantly, $P < 0.001$) inhibiting the effect of TNF-α in the expression of ICAM-1 adhesion molecules in HUVECs.

<u>Example 9 - Assessment of the preventive and curative action of skin irritation in vivo</u>

This study aimed at assessing the *Passiflora alata* extract incorporated into a cosmetic formulation at different concentrations, in the preventive and curative treatment of skin irritation caused by the application of a chemical insult performed in the nasolabial fold region, using the pain scale for subjective assessment. It is known that the irritation by chemical insult is transient, but it is characterized by a micro-inflammatory process with the start of signaling cascades for pain and irritation (erythema, redness).

Example 9.1

The following sample of *Passiflora alata* extract according to the present invention was used:

Sample C (content of total flavonoids 16.46% and content of vitexin-2-o-r 11.87%)

This extract was incorporated into a cosmetic formulation at the concentrations of 0.25%; 0.4%; 0.75% and

1%. The formulations containing the extract were compared with each other, as well as with the placebo (cosmetic formulation without the active principle) and a positive control (dermatological use cortisone). The formulation is found in Table 6.

Table 6 - Cosmetic Formulation

| Component | Concentration (%) |
|---|---|
| Demineralized water | 88.25 |
| Alkyl acrylate TR-1 | 0.20 |
| Dicapryl ether | 2.00 |
| Cyclomethicone D5/D6 VS 7158 | 4.00 |
| Disodium EDTA | 0.10 |
| Cetyl lactate | 1.00 |
| Glyceryl stearate | 0.20 |
| Stearet-2 | 2.10 |
| Stearet-21 | 0.70 |
| Triethanolamine | 0.10 |
| Butylcarbamate iodopropynyl | 0.20 |
| BHT | 0.05 |
| Phenoxyethanol F | 0.90 |
| Xanthan gum | 0.20 |

Two rectangles of 1.0 x 2.0 cm, the so-called sites, were marked with a surgical pen in the left and right nasolabial fold regions of the volunteers between 20 and 50 years old (average 35 years) having a positive sting test in the nasolabial fold region. The study was planned and conducted according to the determinations of Resolution 196/96 of the National Health Council, under the Guidelines and Standards regulating Research involving Human Beings. The study protocol was approved on July 25, 2006 by the Committee on Research Ethics of the Medical Sciences College of the State University of Campinas, under Written Opinion no. 372/2006. The statistical significance ($p < 0,05$) was checked by the analysis of variance (One-Way ANOVA) followed by a Tukey multiple comparison.

Using a medicine dropper, a drop of solution at 10% lactic acid was applied in each site. Irritation was assessed immediately after the application of lactic acid. Then, the products were applied randomly. After 5 and 10 minutes of application, the irritation formed was assessed. This protocol is directed to assess the curative effect.

In the preventive effect, the application was performed during 3 consecutive days twice a day. On the fourth day, the product was applied and after 30 minutes the chemical insult was performed using a drop of solution at 10% lactic acid on each site. Irritation was assessed immediately after the application of lactic acid, after 5 minutes and 10 minutes.

A numeric quantification scale was used to assess the irritation in function of pain intensity and severity in a scale of 0 (absence of pain and irritation) to 10 (intense pain and irritation).

The results of the curative and preventive effects of the treatment are expressed in the graphs of Figures 16 and 17, respectively

In the curative effect, all the cosmetic formulations containing sample C showed the same performance as the commercial corticoid of topical use ($p > 0.05$). The placebo formulation was significantly lower than the corticoid and all the formulations containing the test extract ($p < 0.05$). This data shows that the cosmetic formulations containing *Passiflora alata ex*tracts in any of the concentrations tested are effective in reducing the discomfort sensation (pain, irritation and itching). Their action does not significantly differ from the one presented by the corticoid of topical use and is superior to that of the placebo formulation. Therefore, the active principles present in the *Passiflora alata* extract are responsible for the performance observed in these cosmetic formulations with regard to the reduction of irritation and pain.

In the preventive effect, all the cosmetic formulations containing sample C showed a reduction in the intensity of pain and irritation. These effects were essentially identical to those observed by the corticoid of topical use utilized in the study. The placebo formulation is significantly different from the corticoid ($p < 0.05$), confirming that the efficacy in the prevention of pain and irritation of the cosmetic formulations containing the *Passiflora alata* extract is due to the

presence of sample C therein.

## Claims

**1.** A process for preparing a plant extract of *Passiflora alata,* **characterised by** comprising the steps of:-

a) submitting leaves of *Passiflora alata* plants to an extraction with water to obtain an aqueous extract;
b) submitting the aqueous extract obtained in (a) to at least one elution with a hydroalcoholic solution in a chromatographic column filled with a resin consisting of polyvinylpyrrolidone or polystyrene polymers;
c) concentrating and spray drying the extract obtained in (b).

**2.** The process according to claim 1, **characterised in that** between steps b) and c) it further comprises the steps of:-

- resuspending the product obtained in (b) in hot ethanol and later cooling.

**3.** A cosmetic composition comprising, as an anti-inflammatory active ingredient, a plant extract of the *Passiflora alata* species, said plant extract of *Passiflora alata* being an extract obtained through a process as defined in claim 1 or claim 2.

**4.** A cosmetic composition according to claim 3, **characterised in that** it is an anti-ageing or anti-wrinkle composition.

**5.** A cosmetic composition according to claim 3 or 4, **characterised in that** the plant extract of *Passiflora alata* is present at an amount of 0.25% to 1.0%, by weight, based on the total weight of the composition.

**6.** A pharmaceutical composition comprising a plant extract of the *Passiflora alata* species, said plant extract of *Passiflora alata* being an extract obtained through a process such as defined in claim 1 or claim 2, for use as an anti-inflammatory.

**7.** A pharmaceutical composition for use as an anti-inflammatory according to claim 6, **characterised in that** the plant extract of *Passiflora alata* is present at an amount of 0.25% to 1.0%, by weight, based on the total weight of the composition

**8.** A pharmaceutical composition for use as an anti-inflammatory according to claim 6 or 7, **characterised in that** it is a composition for use by topical administration.

**9.** A pharmaceutical composition for use as an anti-inflammatory according to claim 6 or 7, **characterised in that** it is a composition for use by oral administration.

**10.** A plant extract of *Passiflora alata* for use as an anti-inflammatory agent in cosmetic or pharmaceutical compositions, **characterised in that** said plant extract of *Passiflora alata* is obtained according to the process defined in claim 1 or claim 2.

**11.** A method of reducing the appearance of ageing or of reducing wrinkles, which comprises the use of a cosmetic composition according to any one of claims 3 to 5.

**12.** An extract of *Passiflora alata,* preparable according to the process defined in claim 1 or claim 2.

**13.** An extract of *Passiflora alata* according to claim 12, for use as an anti-inflammatory and analgesic.

**14.** An extract of *Passiflora alata* according to claim 12 or for use as an anti-inflammatory and analgesic according to claim 13, for topical or oral administration.

**15.** An extract of *Passiflora alata* for use as an anti-inflammatory and analgesic, by topical or oral administration.

## Patentansprüche

1. Prozess zum Zubereiten eines Pflanzenextrakts von *Passiflora alata,* **gekennzeichnet durch** die folgenden Schritte:

   a) Unterwerfen von Blättern von *Passiflora alata*-Pflanzen einer Extraktion mit Wasser, um ein wässriges Extrakt zu erhalten;
   b) Unterwerfen des in (a) erhaltenen wässrigen Extrakts wenigstens einer Elution mit einer alkoholisch-wässrigen Lösung in einer Chromatographiesäule, die mit einem Harz gefüllt ist, das aus Polyvinylpyrrolidon- oder Polystyrol-Polymeren besteht;
   c) Konzentrieren und Sprühtrocknen des in (b) erhaltenen Extrakts.

2. Prozess nach Anspruch 1, **dadurch gekennzeichnet, dass** er zwischen den Schritten b) und c) die folgenden weiteren Schritte umfasst:

   - erneutes Suspendieren des in (b) erhaltenen Produkts in heißem Ethanol und später Abkühlen.

3. Kosmetische Zusammensetzung, die als einen entzündungshemmenden aktiven Bestandteil ein Pflanzenextrakt der *Passiflora alata*-Art enthält, wobei das Pflanzenextrakt aus *Passiflora alata* ein Extrakt ist, das durch einen Prozess nach Anspruch 1 oder nach Anspruch 2 erhalten wird.

4. Kosmetische Zusammensetzung nach Anspruch 3, **dadurch gekennzeichnet, dass** es eine Anti-Alterungs- oder Antifalten-Zusammensetzung ist.

5. Kosmetische Zusammensetzung nach Anspruch 3 oder 4, **dadurch gekennzeichnet, dass** das Pflanzenextrakt von *Passiflora alata* in einer Menge im Bereich von 0,25 bis 1,0 Gew.-% bezogen auf das Gesamtgewicht der Zusammensetzung vorhanden ist.

6. Pharmazeutische Zusammensetzung, die ein Pflanzenextrakt der *Passiflora alata*-Art enthält, wobei das Pflanzenextrakt aus *Passiflora alata* ein Extrakt ist, das durch einen Prozess nach Anspruch 1 oder Anspruch 2 erhalten wird und als Entzündungshemmer verwendet wird.

7. Pharmazeutische Zusammensetzung für die Verwendung als Entzündungshemmer nach Anspruch 6, **dadurch gekennzeichnet, dass** das Pflanzenextrakt von *Passiflora alata* in einer Menge im Bereich von 0,25 bis 1,0 Gew.-% in Bezug auf das Gesamtgewicht der Zusammensetzung vorhanden ist.

8. Pharmazeutische Zusammensetzung für die Verwendung als Entzündungshemmer nach Anspruch 6 oder 7, **dadurch gekennzeichnet, dass** es eine Zusammensetzung für die Verwendung durch lokale Verabreichung ist.

9. Pharmazeutische Zusammensetzung für die Verwendung als Entzündungshemmer nach Anspruch 6 oder 7, **dadurch gekennzeichnet, dass** es eine Zusammensetzung für die Verwendung durch orale Verabreichung ist.

10. Pflanzenextrakt aus *Passiflora alata* für die Verwendung als entzündungshemmender Wirkstoff in kosmetischen oder pharmazeutischen Zusammensetzungen, **dadurch gekennzeichnet, dass** das Pflanzenextrakt von *Passiflora alata* gemäß dem Prozess nach Anspruch 1 oder Anspruch 2 erhalten wird.

11. Verfahren zum Verringern der Erscheinung einer Alterung oder zum Verringern von Falten, das die Verwendung einer kosmetischen Zusammensetzung nach einem der Ansprüche 3 bis 5 umfasst.

12. Extrakt von *Passiflora alata,* das gemäß dem Prozess nach Anspruch 1 oder Anspruch 2 zubereitet werden kann.

13. Extrakt von *Passiflora alata* nach Anspruch 12 für die Verwendung als Entzündungshemmer und Analgetikum.

14. Extrakt von *Passiflora alata* nach Anspruch 12 oder für die Verwendung als ein Entzündungshemmer und Analgetikum nach Anspruch 13 für lokale oder orale Verabreichung.

15. Extrakt von *Passiflora alata* für die Verwendung als Entzündungshemmer und Analgetikum durch lokale oder orale Verabreichung.

## Revendications

**1.** Procédé de préparation d'un extrait végétal de *Passiflora alata,* **caractérisé par le fait qu'**il comprend les étapes consistant à :-

a) soumettre des feuilles de plantes de *Passiflora alata* à une extraction avec de l'eau pour obtenir un extrait aqueux ;
b) soumettre l'extrait aqueux obtenu en (a) à au moins une élution avec une solution hydroalcoolique dans une colonne chromatographique remplie d'une résine constituée de polymères de polyvinylpyrrolidone ou de polystyrène ;
c) concentrer et sécher par atomisation l'extrait obtenu en (b).

**2.** Procédé selon la revendication 1, **caractérisé en ce que**, entre les étapes b) et c), il comprend en outre les étapes consistant à :-

- remettre en suspension le produit obtenu en (b) dans de l'éthanol chaud puis le laisser refroidir.

**3.** Composition cosmétique comprenant, en tant que substance active anti-inflammatoire, un extrait végétal de l'espèce *Passiflora alata,* ledit extrait végétal de *Passiflora alata* étant un extrait obtenu par un procédé tel que défini dans la revendication 1 ou la revendication 2.

**4.** Composition cosmétique selon la revendication 3, **caractérisée en ce qu'**il s'agit d'une composition antivieillissement ou antiride.

**5.** Composition cosmétique selon la revendication 3 ou 4, **caractérisée en ce que** l'extrait végétal de *Passiflora alata* est présent en une quantité de 0,25 % à 1,0 %, en poids, par rapport au poids total de la composition.

**6.** Composition pharmaceutique comprenant un extrait végétal de l'espèce *Passiflora alata,* ledit extrait végétal de *Passiflora alata* étant un extrait obtenu par un procédé tel que défini dans la revendication 1 ou la revendication 2, pour utilisation en tant qu'anti-inflammatoire.

**7.** Composition pharmaceutique pour utilisation en tant qu'anti-inflammatoire selon la revendication 6, **caractérisée en ce que** l'extrait végétal de *Passiflora alata* est présent en une quantité de 0,25 % à 1,0 %, en poids, par rapport au poids total de la composition

**8.** Composition pharmaceutique pour utilisation en tant qu'anti-inflammatoire selon la revendication 6 ou 7, **caractérisée en ce qu'**il s'agit d'une composition pour utilisation par administration topique.

**9.** Composition pharmaceutique pour utilisation en tant qu'anti-inflammatoire selon la revendication 6 ou 7, **caractérisée en ce qu'**il s'agit d'une composition pour utilisation par administration orale.

**10.** Extrait végétal de *Passiflora alata* pour utilisation en tant qu'agent anti-inflammatoire dans des compositions cosmétiques ou pharmaceutiques, **caractérisé en ce que** ledit extrait végétal de *Passiflora alata* est obtenu selon le procédé défini dans la revendication 1 ou la revendication 2.

**11.** Procédé de réduction de l'apparition du vieillissement ou de réduction des rides, qui comprend l'utilisation d'une composition cosmétique selon l'une quelconque des revendications 3 à 5.

**12.** Extrait de Passiflora *alata,* pouvant être préparé selon le procédé défini dans la revendication 1 ou la revendication 2.

**13.** Extrait de Passiflora *alata* selon la revendication 12, pour utilisation en tant qu'anti-inflammatoire et analgésique.

**14.** Extrait de Passiflora *alata* selon la revendication 12 ou pour utilisation en tant qu'anti-inflammatoire et analgésique selon la revendication 13, pour administration topique ou orale.

**15.** Extrait de Passiflora *alata* pour utilisation en tant qu'anti-inflammatoire et analgésique par administration topique ou orale.

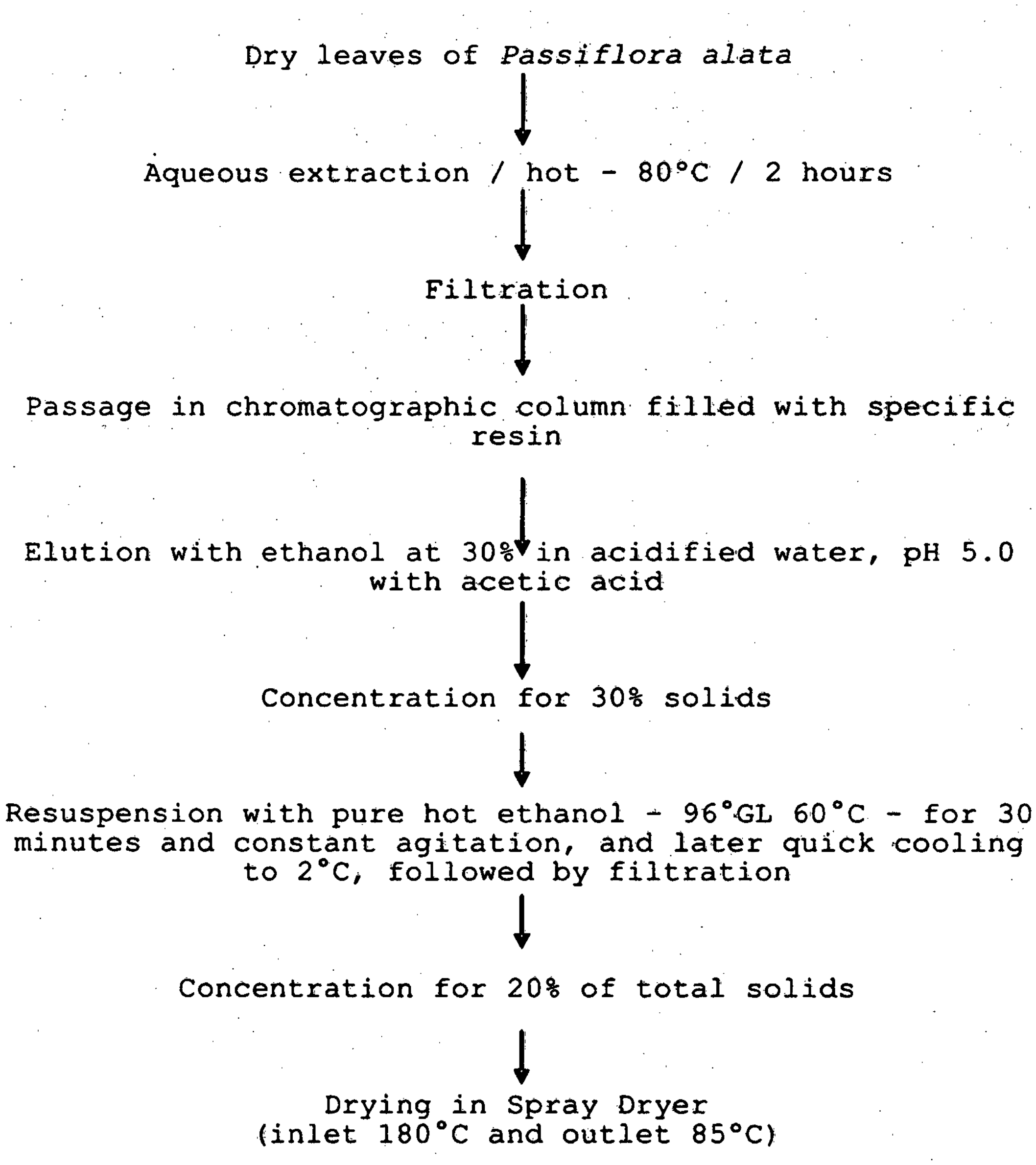
Dry leaves of *Passiflora alata*
Aqueous extraction / hot - 80°C / 2 hours
Filtration
Passage in chromatographic column filled with specific resin
Elution with ethanol at 30% in acidified water, pH 5.0 with acetic acid
Concentration for 30% solids
Resuspension with pure hot ethanol - 96°GL 60°C - for 30 minutes and constant agitation, and later quick cooling to 2°C, followed by filtration
Concentration for 20% of total solids
Drying in Spray Dryer (inlet 180°C and outlet 85°C)

FIGURE 1

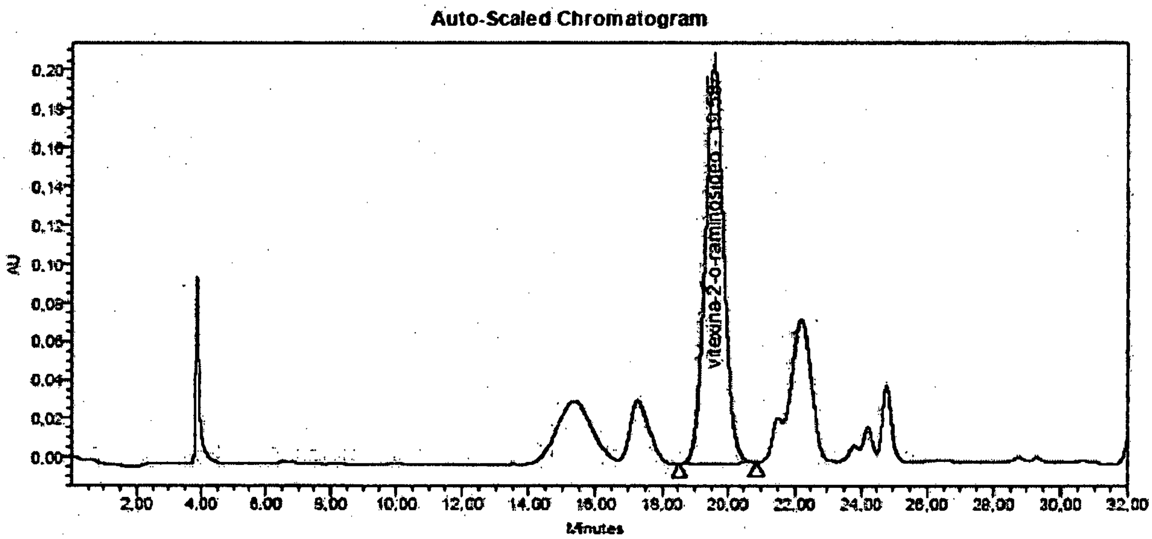
Auto-Scaled Chromatogram
AU
0,00
0,02
0,04
0,06
0,08
0,10
0,12
0,14
0,16
0,18
0,20
vitexina-2-o-raminosideo
2,00
4,00
6,00
8,00
10,00
12,00
14,00
16,00
18,00
20,00
22,00
24,00
26,00
28,00
30,00
32,00
Minutes

FIGURE 2

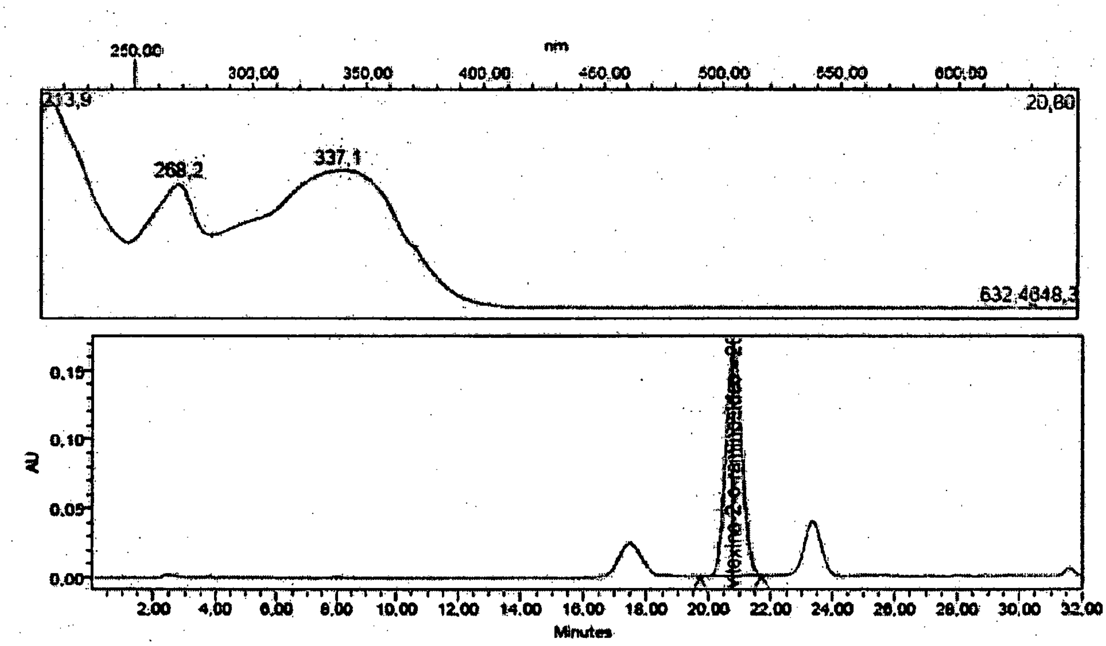
nm
250,00
300,00
350,00
400,00
450,00
500,00
550,00
600,00
213,9
268,2
337,1
20,80
632,4848,3
AU
0,15
0,10
0,05
0,00
2,00
4,00
6,00
8,00
10,00
12,00
14,00
16,00
18,00
20,00
22,00
24,00
26,00
28,00
30,00
32,00
Minutes

FIGURE 3

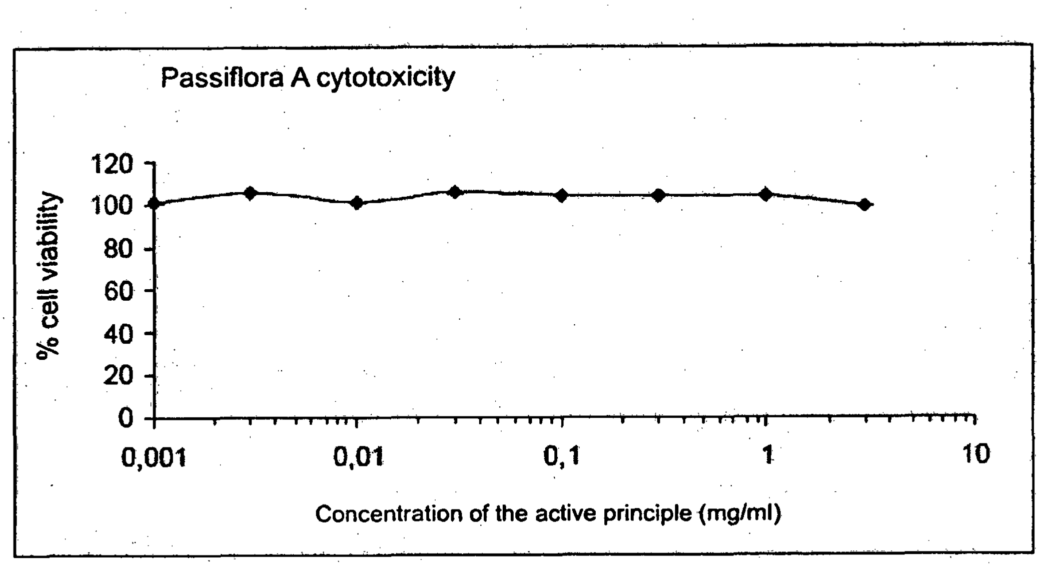


FIGURE 4
Cytotoxicity of the Test Sample in 3T3 Cells

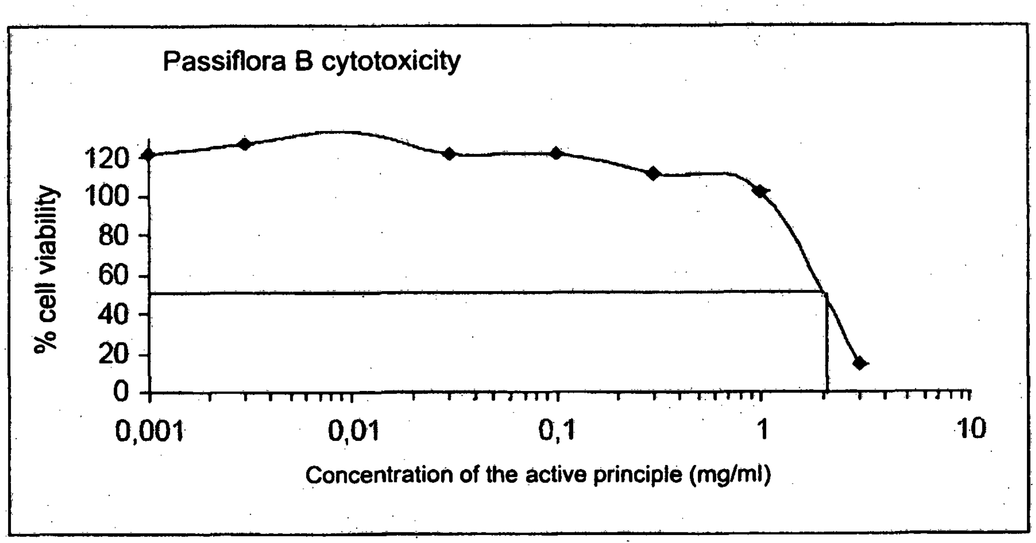


FIGURE 5
Cytotoxicity of the Test Sample in 3T3 Cells

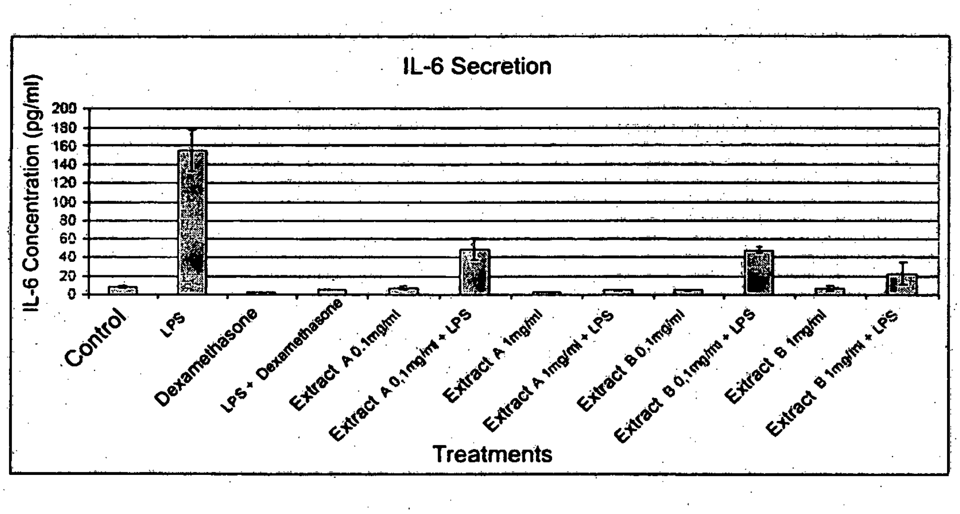


FIGURE 6

Analysis of IL-6 Secretion by Human Fibroblasts

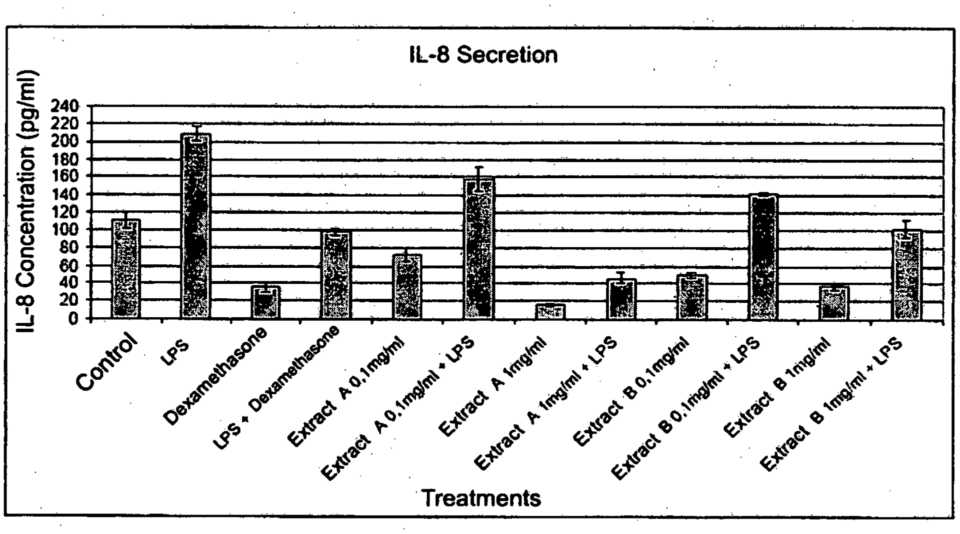


FIGURE 7

Analysis of IL-8 Secretion by Human Fibroblasts.

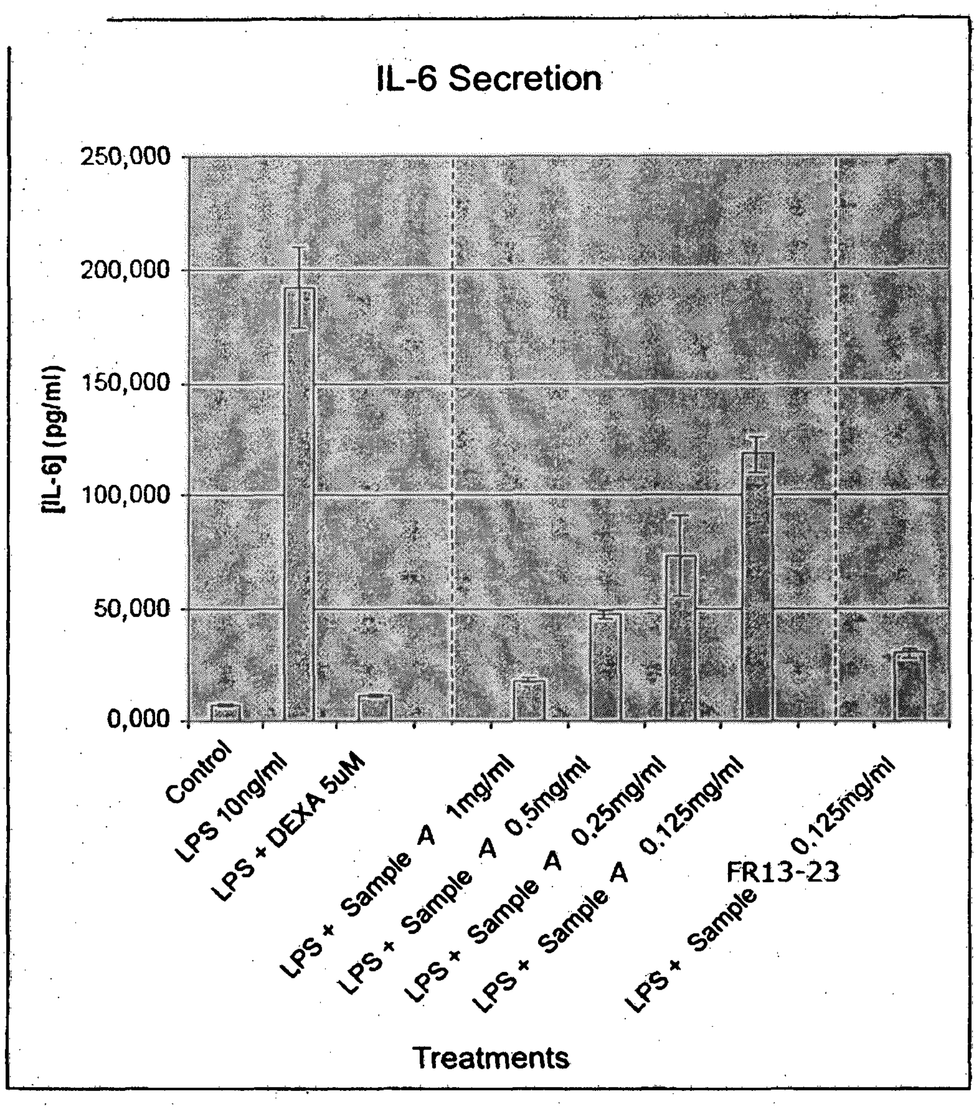


FIGURE 8

Analysis of IL-6 Secretion by Human Fibroblasts

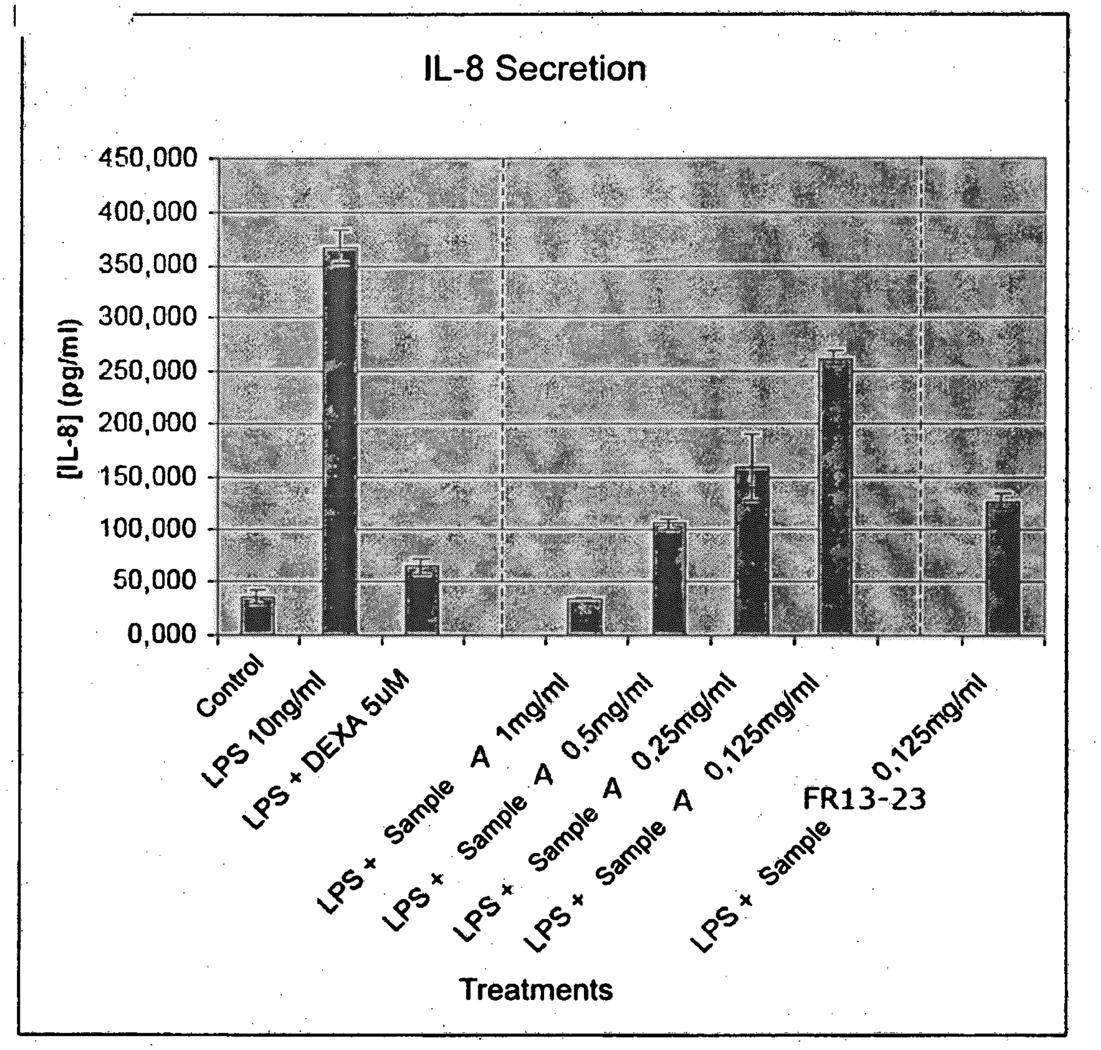


FIGURE 9

Analysis of IL-8 Secretion by Human Fibroblasts

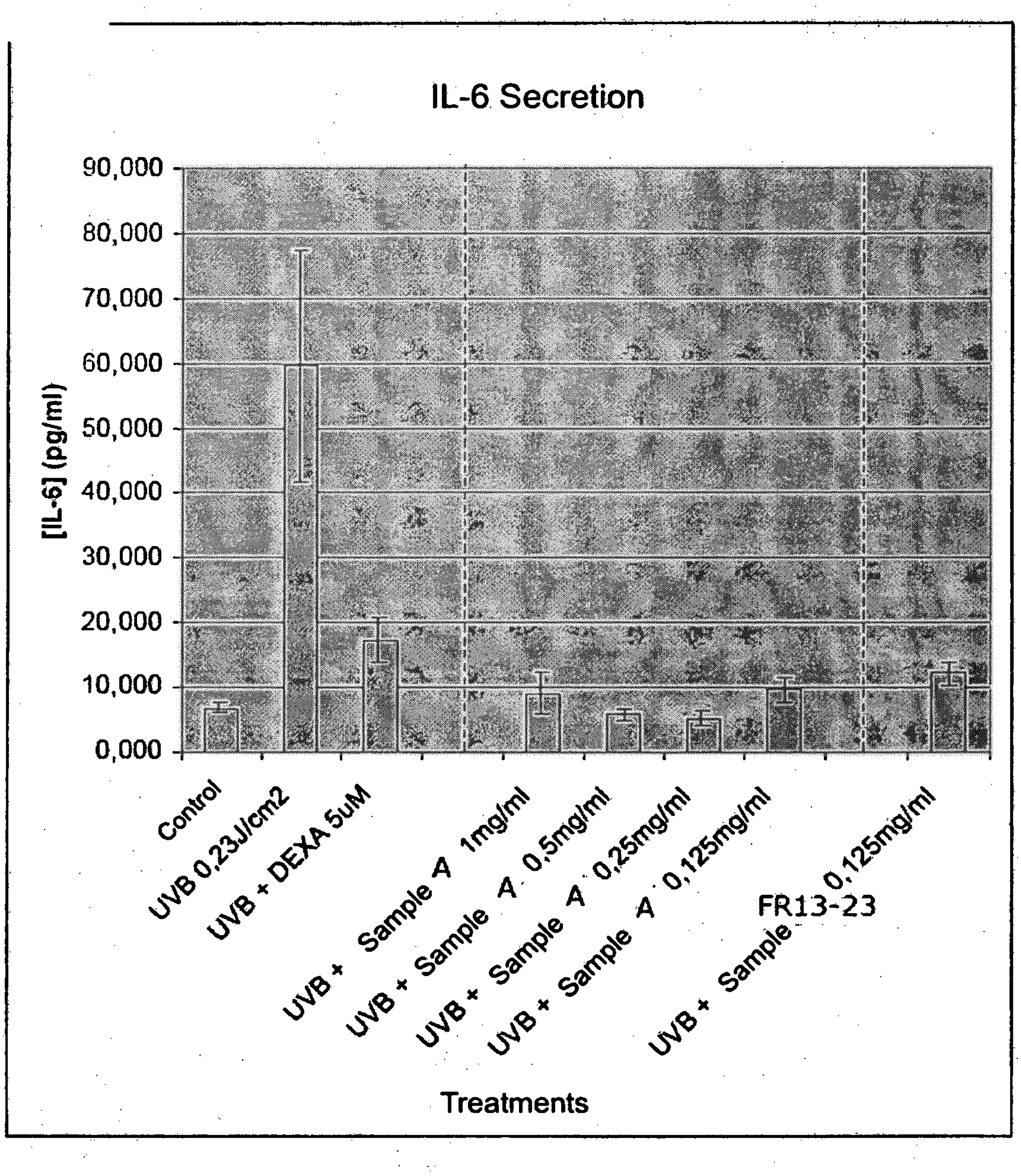


FIGURE 10

Analysis of IL-6 Secretion by Human Fibroblasts

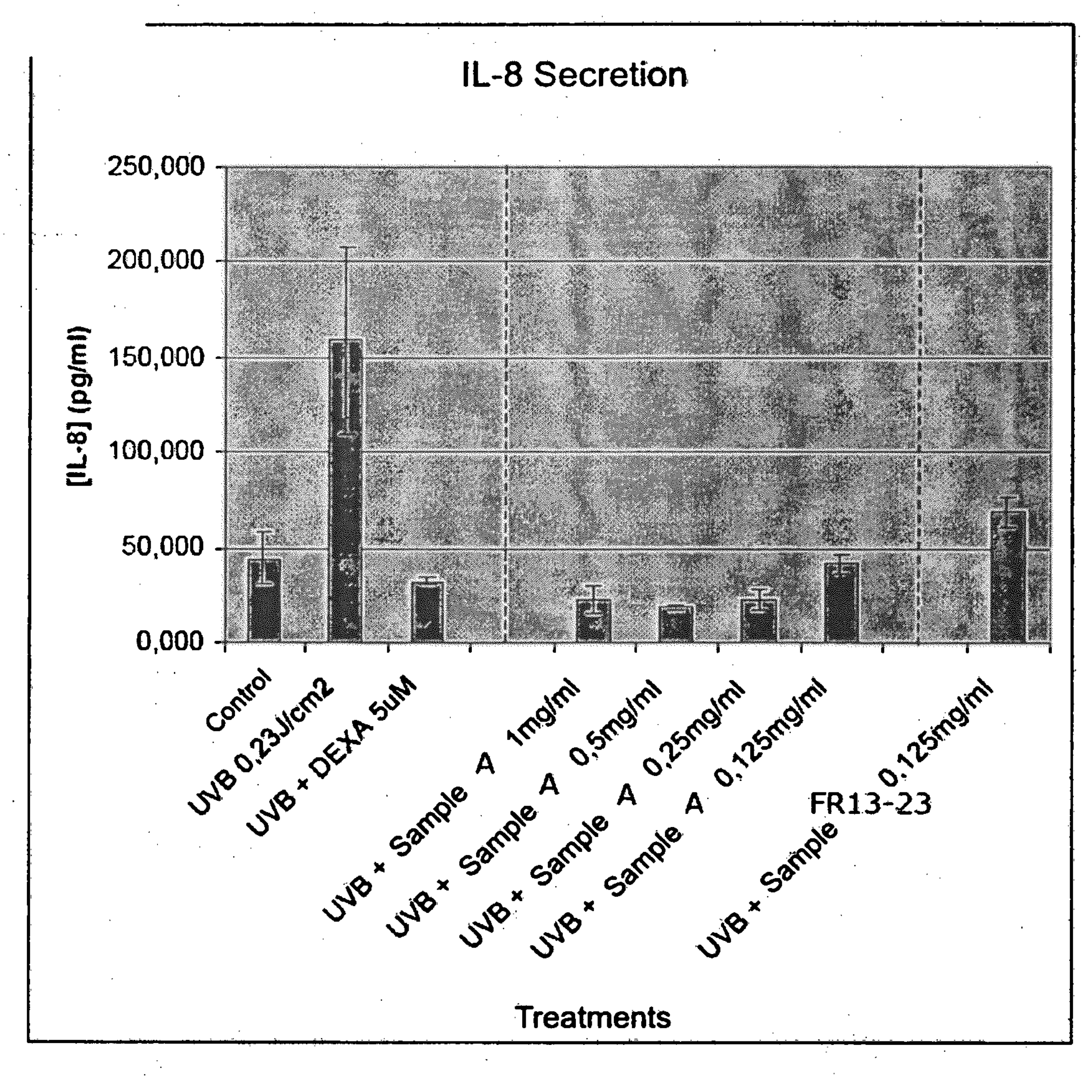


FIGURE 11

Analysis of IL-8 Secretion by Human Fibroblasts.

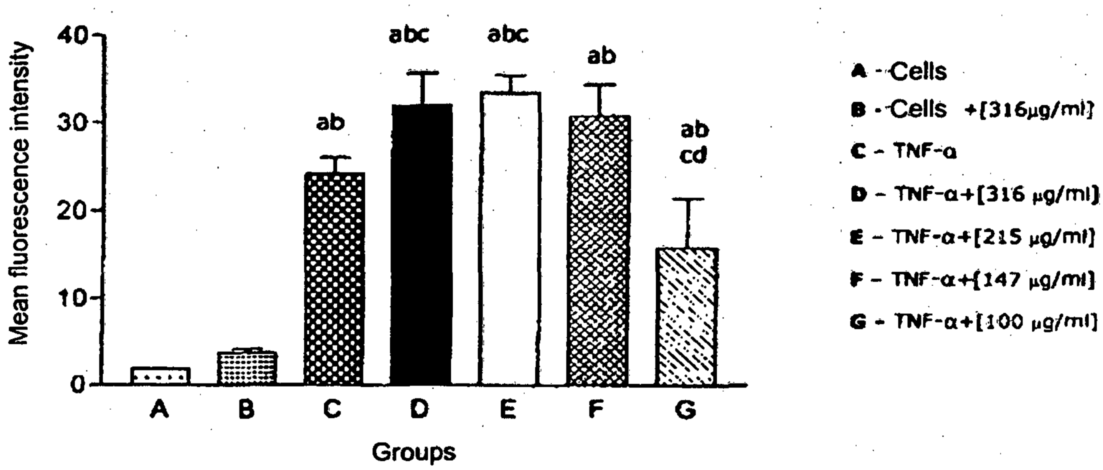


FIGURE 12

Expression of ICAM-1 adhesion molecules on the surface of HUVEC cells.
The statistical significance is indicated by letters

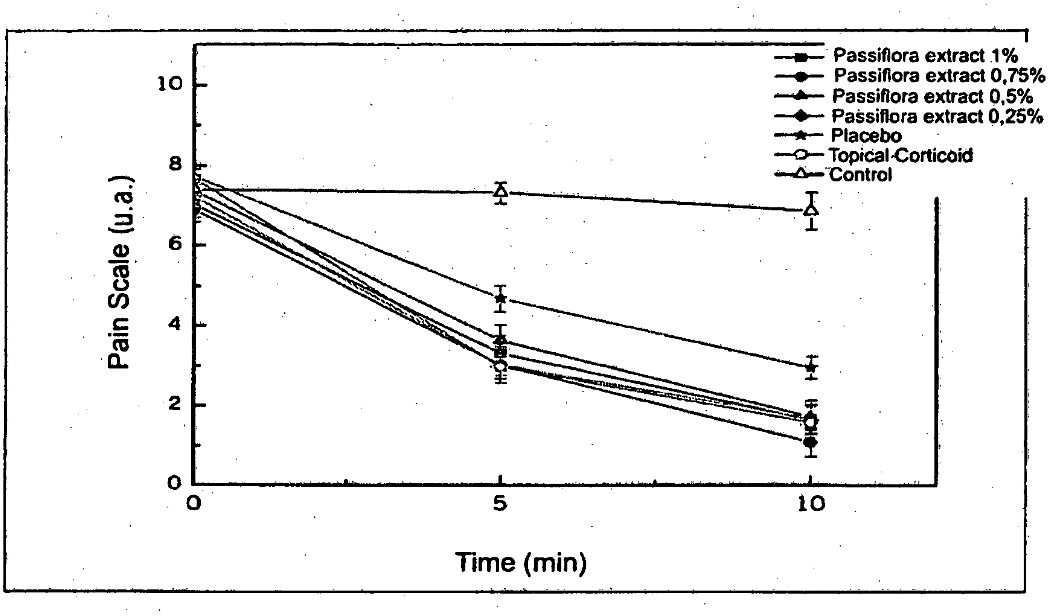


FIGURE 13

Evolution of the curative effect of products in time.
Mean Error (n= 30 for each product)

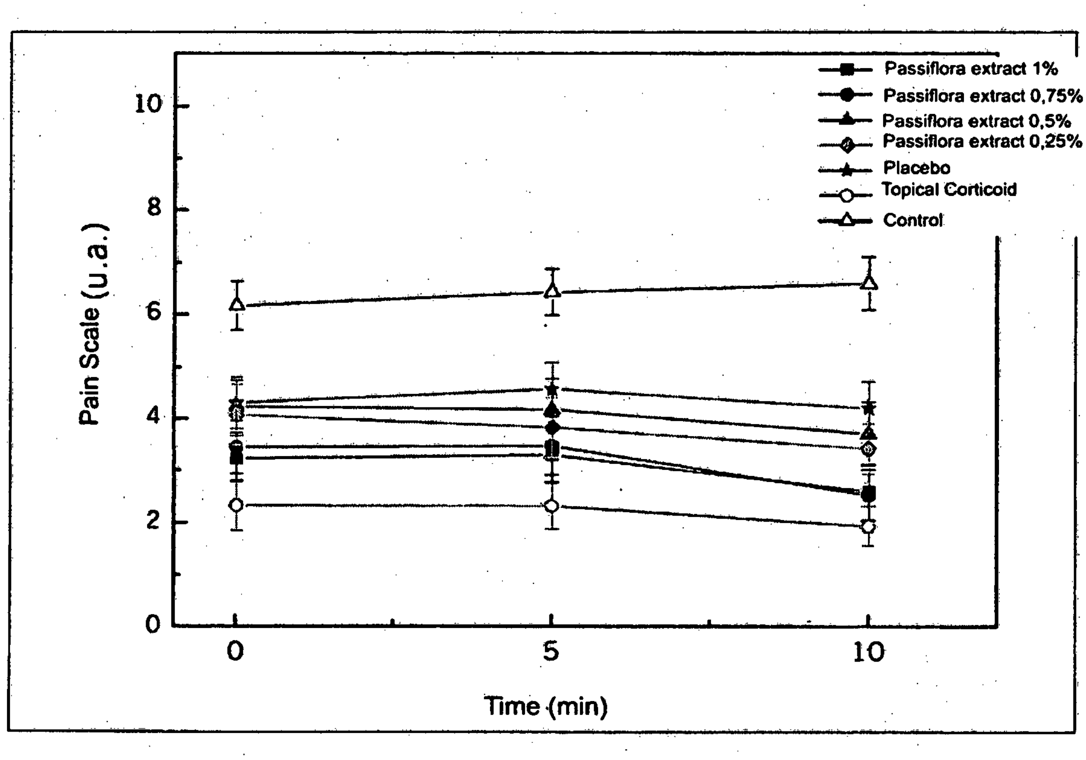


FIGURE 14

Evolution of the preventive effect of products in time.
Mean Error (n= 30 for each product)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005097153 A
- EP 1537789 A
- JP 200200336 B
- JP 2000159657 B
- JP 2001122731 B
- JP 2002332224 B
- JP 7233044 B
- WO 2005077328 A
- JP 2001226219 B
- JP 7118139 B
- JP 2004155664 B
- WO 2005053435 A


### Non-patent literature cited in the description


- Guidance Document on Using In Vitro Data to Estimate In Vivo Starting Doses for Acute Toxicity. NIH Publication 01-4500, 2001
- ZEBET/ECVAM/COLIPA Standard Operating Procedure. *In Vitro 3T3 Phototoicity Test,* 26 April 1998
- **SPIELMANN H ; LIEBSCH M ; DORING B ; MOLDENHAUER F.** First results of an EC/COLIPA validation project of in vitro phototoxicity testing methods. *ALTEX,* 1994, vol. 11 (1), 22-31
- **MARON ; AMES.** *OECD Guidelines,* 1997